(19) Europäisches Patentamt
European Patent Office
Office européen des brevets

(11) **EP 4 276 841 A1**

(12) **EUROPEAN PATENT APPLICATION**

(43) Date of publication:
**15.11.2023  Bulletin 2023/46**

(51) International Patent Classification (IPC):
**G16H 10/60** (2018.01)          **G16H 50/30** (2018.01)
**G16H 40/20** (2018.01)

(21) Application number: **22172780.3**

(22) Date of filing: **11.05.2022**

(52) Cooperative Patent Classification (CPC):
**G16H 10/60; G16H 40/20; G16H 50/30**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Siemens Healthcare GmbH
91052 Erlangen (DE)**

(72) Inventors:
• **Anandakumar, M.**
  **560017 Bangalore (IN)**
• **GS, Vinay**
  **577414 Shivamogga (IN)**
• **H D, Chetan**
  **560085 Bengaluru (IN)**
• **K, Karthik**
  **583103 Hospet (IN)**
• **Kedlaya, Nitheesh**
  **560040 Bangalore (IN)**
• **Shenoy, Raghavendra**
  **560068 Bangalore (IN)**

(54) **METHOD AND SYSTEM FOR ANALYSING EFFICIENTLY PATIENT DATA**

(57)     A computer-implemented method and system for analysing efficiently patient data of patients comprising the steps of: loading (S1) by a user-client device (5) of a health investigating user (U) at least one patient data record, PDREC, of each patient (P) forming part of a selected group of patients (P) assigned to the client device (5) of the respective health investigating user (U) for medical investigation of the assigned group of patients (P) from a transitionary database (2) of a health data processing system (1), wherein each loaded patient data record, PDREC, of a patient (P) of said assigned group of patients comprises measured parameter values of a selected group of predefined vital health related parameters, vp, measured for the respective patient (P) by means of medical devices (3) during previously performed medical investigations of the respective patient (P); determining (S2) by a processor of the user-client device (5) of the health investigating user (U) an extent of deviations of parameter values of the predefined vital health related parameters, vp, within each loaded patient data record, PDREC, of a patient from reference parameter values; and sorting (S3) by the processor of the user-client device (5) of the health investigating user (U) the loaded patient data records, PDRECs, of the group of patients (P) assigned to the health investigating user (U) according to patient list indices to generate automatically a sorted patient record list, SPRL, of the assigned patients (P) depending on the determined extent of deviations of the predefined vital health related parameters, vp, and/or depending on a determined number of vital health related parameters falling out of range for performing a data analysis of patient data included in the patient data records, PDRECs, of the generated sorted patient record list, SPRL.

## FIG 2

**Description**

The invention relates to a computer-implemented method and system for analysing efficiently patient data of a plurality of patients.

For investigation of the health of a patient, a health investigating user such as a physician has to evaluate different health related parameters measured for the investigated patients by means of medical devices. Often, different health investigating users cooperate to investigate the medical state of one or more patients. For example, a patient may be referred by a physician to a cardiologist for further investigating a medical state of the patient's heart. When the cardiologist conducts his own examination of the referred patient, he might suggest additional scans to get a deeper understanding of the medical state of the patient at hand, in particular to eliminate false assumptions concerning the health of the patient. For this, the patient is referred by the cardiologist to a radiologist to perform the necessary scans. Once the patient has been scanned by a medical scanning device of the radiologist, the generated images can be reviewed by the radiologist and inferences or observations made by the radiologist are normally documented in region of interest (ROI) reports. After the images have been read and reviewed by the radiologist they can be then made be available to the cardiologist in charge to make necessary decisions concerning the treatment of the investigated patient.

A health investigating user such as a cardiologist normally has to evaluate patient data records of a plurality of patients assigned to the cardiologist each day. Accordingly, it is a challenge for the cardiologist to prioritize and order the received patient data of a plurality of patients to decide what patient data record requires the cardiologist's immediate attention. However, in a conventional system, it is difficult for a cardiologist to quickly infer criticality of a medical situation or state of a patient since the data pertaining to various health related parameters are spread across different data fields in a patient data record of a patient and health relevant information is spread across various images taken of the respective patient by the same or different medical devices operated by other health investigating users such as radiologists. As a consequence, a cardiologist has to go normally through the entire details of the patient data records to even arrive to a group of patients that requires his immediate attention. Further, this conventional selection requires the cardiologist to make mental and digital or manual notes across the patient data records of all patients assigned to the cardiologist to sort them based on the observed differences and variations of health related parameters deemed by the cardiologist to be relevant for the medical health state of the investigated patients. Accordingly, a conventional selection process of patients who require the immediate attention of the cardiologist is time-consuming unreliable and inefficient. Moreover, this heuristic approach is error-prone and may have as a consequence that patients in a critical health situation may not be investigated immediately by the cardiologist so that precious time is lost. This may lead even to a situation where patients in a very critical health state may not be investigated by the cardiologist in time with severe consequences for the respective patients.

Accordingly, it is an object of the present invention to provide a method and system which allows to analyse patient data of a plurality of patients by a health investigating user more efficiently and to reduce the time required for medical investigation of patients.

This object is achieved according to a first aspect of the present invention by a computer-implemented method comprising the features of claim 1.

The invention provides according to a first aspect a computer-implemented method for analysing efficiently patient data of patients comprising the steps of:

> loading by a user-client device of a health investigating user at least one patient data record of each patient forming part of a selected group of patients assigned to the client device of the respective health investigating user for medical investigation of the assigned group of patients from a transitionary database of a health data processing system, wherein each loaded patient data record of a patient of said assigned group of patients comprises measured parameter values of a selected group of predefined vital health related parameters measured for the respective patient by means of medical devices during previously performed medical investigations of the respective patient;
> determining by a processor of the user-client device of the health investigating user an extent of deviations of parameter values of the predefined vital health related parameters within each loaded patient data record of a patient from reference parameter values; and
> sorting by the processor of the user-client device of the health investigating user the loaded patient data records of the group of patients assigned to the client device of the health investigating user according to patient list indices to generate automatically a sorted patient record list of the assigned patients depending on the determined extent of deviations of the predefined vital health related parameters and/or depending on a determined number of vital health related parameters falling out of range for performing a data analysis of patient data included in the patient data records of the generated sorted patient record list.

The data analysis can in possible embodiment be performed or supported by a diagnostic medical software tool used to perform data analysis of the patient data. This medical software tool can be executed on the client device of the health investigating user or on server connected to the client device of the health investigating user though a data network.

In a possible embodiment of the computer-implemented method according to the first aspect of the present invention the reference parameter values comprise configurable set parameter values or configurable limits of parameter value ranges of the vital health related parameters and/or comprise benchmark parameter values of the vital health related parameters derived from parameter values of vital health related parameters read from other loaded patient data records of the same patient.

The configurable set parameters or configurable limits of parameter values are disease specific. The computer-implemented method according to the present invention can be used for different diseases.

In a possible embodiment of the computer-implemented method according to the first aspect of the present invention, a group of disease specific and/or region specific and/or investigation specific vital health related parameters being critical for a health condition of a patient is selected from available health related parameters, wherein associated configurable reference parameter values or configurable limits of the selected specific vital health related parameters are adjusted or reconfigured in a configuration process by a health system administrator of the health data processing system.

In a still further possible embodiment of the computer-implemented method according to the first aspect of the present invention, for each vital health related parameter, a vital parameter criticality metric ,vp-cm, is automatically determined by a processor of the client device of the health investigating user depending on the determined extent of deviations of the measured parameter values of the respective vital health related parameter from the associated reference parameter values.

In a further possible embodiment of the computer-implemented method according to the first aspect of the present invention, the determined vital parameter criticality metrics, vp-cm, of all vital health related parameters are processed by the processor of the client device of the health investigating user to calculate a total patient criticality metric ,P-CM, indicating a total criticality of a health condition of the investigated patient.

In a further possible embodiment of the computer-implemented method according to the first aspect of the present invention, the patient list indices used for sorting the loaded patient data records of the selected group of patients assigned to the health investigating user are derived from the calculated total patient criticality metrics, P-CMs, of the respective group of patients.

In a further possible embodiment of the computer-implemented method according to the first aspect of the present invention, the set parameter values of the vital health related parameters comprise adjustable threshold values for parameter value range limits derived from default settings of medical devices used to collect patient data of health related parameters stored in patient data records of patients.

In a further possible embodiment of the computer-implemented method according to the first aspect of the present invention, for performing a medical investigation, the user-client device of an authenticated health investigating user is logged-in for getting access to the parameter values of vital health related parameters included in the patient data records of patients forming part of a selected group of patients assigned to the authenticated health investigating user and being stored in the transitionary database of the health data processing system.

In a still further possible embodiment of the computer-implemented method according to the first aspect of the present invention, the patient data records include patient data comprising measured parameter values of a variety of different vital and non-vital health related parameters, metadata of medical devices used for the measurements of the health related parameters during investigations and personal data of the plurality of patients.

In a still further possible embodiment of the computer-implemented method according to the first aspect of the present invention, the generated sorted patient record list is displayed on a graphical user interface of the client device to the health investigating user.

In a still further possible embodiment of the computer-implemented method according to the first aspect of the present invention, the measured parameter values of health related parameters comprise image data and/or audio data captured by sensors of medical devices.

In a still further possible embodiment of the computer-implemented method according to the first aspect of the present invention, the measured parameter values of vital health related parameters of a patient data record displayed on the graphical user interface of the client device of the health investigating user are displayed to on a display unit of the client device of the health investigating user in a pop-up window if the respective patient data record listed in the sorted patient record list is selected by the health investigating user.

In a still further possible embodiment of the computer-implemented method according to the first aspect of the present invention, if the total patient criticality metric ,P-CM, of a patient calculated by the processor of the client device of the health investigating user indicates an acute critical health condition of the respective patient, the generation of a corresponding warning message is automatically triggered and displayed on the graphical user interface of the local client device of the health investigating user and/or transmitted in a messaging subsystem of the health data processing system to remote client devices of other users, in particular health investigating users.

In a further possible embodiment of the computer-implemented method according to the first aspect of the present invention, the parameter values of the health related parameters are measured by portable or by non-portable medical devices.

In a further possible embodiment of the computer-implemented method according to the first aspect of the present invention, the parameter values of the health related parameters are measured by sensors of wearable medical devices attached to patients or carried by the respective patients.

In a possible embodiment of the computer-implemented method the data analysis of patient data included in selected patient data records, PDECs, of the generated sorted patient record list, SPRL, is performed by means of at least one matching medical software tool executed on a client device (of a health investigating user and/or by a server of the health data processing system.

The invention provides according to a further aspect a client device of a health investigating user comprising the features of claim 15.

The invention provides according to the second aspect a client device of a health investigating user, said client device having a data interface used to load at least one patient data record of each patient forming part of a selected group of patients assigned to the respective health investigating user for medical investigation of the assigned group of patients from a transitionary database of a health data processing system, wherein each loaded patient data record of a patient of said assigned group of patients comprises measured parameter values of vital health related parameters measured for the respective patient by means of medical devices during previously performed medical investigations of the respective patient and having a data processing unit used to calculate an extent of deviations of parameter values of a selected group of predefined vital health related parameters within each loaded patient data record of a patient from reference parameter values, and configured to sort the loaded patient data records of the group of patients assigned to the health investigating user according to patient list indices to generate automatically a sorted patient record list of the assigned patients depending on the calculated extent of deviations of the predefined vital health related parameters for performing a data analysis of patient data included in the patient data records of the generated sorted patient record list displayed on a graphical user interface of the client device to the health investigating user and/or supplied to matching medical software tools.

The invention provides according to a further aspect a health data processing system comprising the features of claim 17.

The invention provides according to a third aspect a health data processing system comprising client devices of health investigating users, in particular physicians, having access to a transitionary database of the health data processing system, wherein client devices are adapted to perform a computer-implemented method according to the first aspect of the present invention.

In a possible embodiment of the health data processing system according to the third aspect of the present invention, a plurality of patient data records of a plurality of patients including measured parameter values of health related parameters are collected by distributed medical devices during investigations of the plurality of patients.

In a possible embodiment of the health data processing system according to the third aspect of the present invention, a scheduler is adapted to process the plurality of patient data records to extract parameter values of vital health related parameters selected from available health related parameters by means of a terminal of a health system administrator of the health data processing system and to store the extracted parameter values of the vital health related parameters in the transitionary database of the health data processing system.

In the following, possible embodiments of the different aspects of the present invention are described in more detail with reference to the enclosed figures.

Fig. 1      shows a schematic diagram for illustrating a possible exemplary embodiment of a health data processing system according to an aspect of the present invention;

Fig. 2      shows a flowchart for illustrating the main steps of a computer-implemented method for analysing efficiently patient data of patients according to an aspect of the present invention;

Fig. 3      shows heart conditions to illustrate a possible use case of a computer-implemented method and system according to the present invention;

Fig. 4      shows a diagram for illustrating an example for a vital health related parameter used by the method and system according to the present invention;

Figs. 5A, 5B      illustrate a possible workflow implementing the computer-implemented method and system according to the present invention;

Fig. 6      illustrates a possible implementation for an input mask used by a system administrator to configure vital health related parameters;

Fig. 7      shows an example of a sorted patient record list generated by a computer-implemented method and system according to the present invention;

Fig. 8      shows an example of a pop-up window displayed on a screen of a graphical user interface to a health investigating user according to a possible implementation of the computer-implemented method and system according to the present invention.

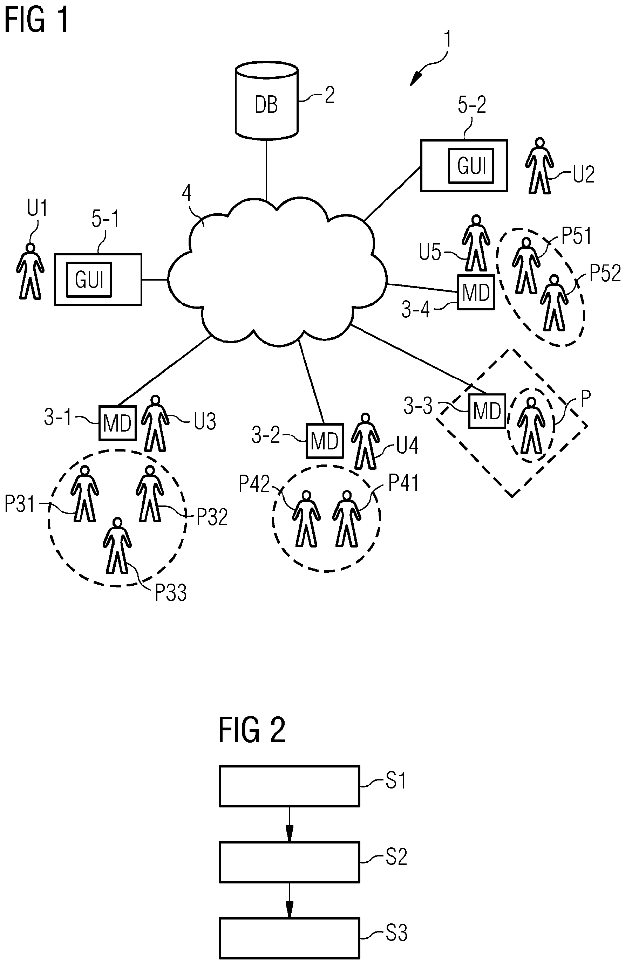

Fig. 1 shows schematically a possible exemplary embodiment of a health data processing system 1 according to an aspect of the present invention. The health data processing system 1 comprises in the illustrated example a transitionary database 2 used for storing patient data and patient data records PDRECs of a plurality of patients P. The patient data can be generated by use of medical devices 3-1, 3-2, 3-3, 3-4 connected via a data network or a cloud 4 to the transitionary database 2 of the health data processing system 1. The medical devices 3-i for instance comprise computer tomographs or magnetic resonators adapted to generate two-dimensional or three-dimensional image data of investigated patients P. The medical devices 3-i can comprise any kind of medical devices to generate patient data reflecting the health condition of the investigated patient P. The medical devices 3-i can also comprise sensors attached to the investigated patient P such as electrodes. Further, the medical devices 3-i can also comprise sensors provided at wearable medical devices 3-i attached to the patients P or carried by the patients P. Each medical device 3 can be operated by an associated user, for instance a technician taking images of body parts of the investigated patient P. For instance, user U3 illustrated in Fig. 1 may take computer tomograph pictures of the investigated patients P31, P32, P33 shown in Fig. 1. The generated patient data can be structured in patient data records of the investigated patients P and uploaded by the radiologist U3 to the transitionary database 2 of the health data processing system 1. Other health investigating users such as cardiologists U1, U2 shown in Fig. 1 may have access to the transitionary database 2 via a corresponding user-client device 5-1, 5-2 as illustrated in Fig. 1. In the illustrated simple example of Fig. 1, the users U1, U2 can be for instance cardiologists investigating the heart condition of investigated patients P assigned to the respective cardiologist. Each cardiologist U1, U2 does investigate the heart condition of a selected group of patients P assigned to the respective cardiologist U1, U2.

Fig. 2 shows a flowchart of a possible exemplary embodiment of a computer-implemented method for analysing efficiently patient data of patients P. In the illustrated embodiment, the computer-implemented method comprises three main steps S1, S2, S3. The computer-implemented method illustrated in the flowchart of Fig. 2 can be executed on a user-client device 5-i of a health investigating user U such as a cardiologist.

In a first step S1, at least one patient data record PDREC of each patient P forming part of a selected group of patients P assigned to the respective health investigating user U for medical investigation of the assigned group of patients P is loaded from the transitionary database 2 of the health data processing system 1. Each loaded patient data record PDREC of a patient P of the assigned group of patients comprises measured parameter values of a selected group of predefined vital health related parameters $v_p$ measured for the respective patient P by means of medical devices 3-i during previously performed medical investigations of the respective patient P. These previously performed medical investigations can for instance comprise investigations performed by a radiologist by means of an image generation medical device 3-i.

In a further step S2, an extent of deviations of parameter values of the predefined vital health related parameters $v_p$ within each loaded patient data record PDREC of a patient P from reference parameter values is determined by a calculation unit of the user-client device 5. The reference parameter values can comprise in a possible embodiment configurable set parameter values or configurable limits of parameter value ranges of the respective vital health related parameters $v_p$. The reference parameter values can also comprise benchmark parameter values of the vital health related parameters $v_p$ derived from parameter values of vital health related parameters $v_p$ read from other loaded patient data records PDRECs of the same patient P or of other patients of a reference patient group.

In a further step S3, the loaded patient data records PDRECs of the group of patients assigned to the health investigating user such as the cardiologist U1 are sorted by the user-client device 5-i according to patient list indices to generate automatically a sorted patient record list SPRL of the assigned patients P depending on the determined extent of deviations of the predefined vital health related parameters $v_p$ and/or depending on weights given to the vital health related parameters for performing a final data analysis of patient data included in the patient data records PDRECs of the generated sorted patient record list SPRL. In a possible embodiment the sorting in step S3 is not just based on deviations from the normal values, but also based on the weights given to the vital health related parameters. These weights may vary significantly based on the deviation, leading to a higher or lower criticality. The vital health related parameters can have different weights. Additionally the weights of each parameter based on the variation of the values is not linear or common across every vital parameter. Due to this characteristic, the total criticality score varies differently for different patients P. The patient list indices used for sorting the loaded patient data records PDRECs of the selected group of patients assigned to the health investigating user U in step S3 can be derived in a possible embodiment from calculated total patient criticality metrics P-CM of the respective group of patients. The total patient criticality metrics P-CM can be calculated by a processor of the user-client device 5-i.

In a possible embodiment, for each vital health related parameters $v_p$, a vital parameter criticality metric vp-cm can be automatically determined by the processor of the client device 5 of the health investigating user or cardiologist U depending on the determined extent of deviations of the measured parameter values of the respective vital health related parameters $v_p$ from the associated reference parameter values. The extent of deviations is determined in step S2 of the computer-implemented method illustrated in the flowchart of Fig. 2. In a further sub step, the determined vital parameter criticality metrics vp-cm of all vital health related parameters $v_p$ are processed by the processor of the client device 5-i of the health investigating user U to calculate the total patient criticality metrics P-CM indicating the total criticality of the health condition of the investigated patient P. The total patient criticality metric P-CM for a respective group of patients assigned to the health investigating user or cardiologist U can form the basis for patient list indices used for sorting the loaded patient data records PDRECs to provide the sorted patient record list SPRL which can be displayed on a screen of the graphical user interface of the client device 5 of the health investigating user.

In a possible embodiment of the computer-implemented method according to the present invention, a group of disease-specific and/or region-specific and/or investigation-specific vital health related parameters $v_p$ being critical for a health condition of a patient P can be preselected from all available health related parameters by a health system administrator of the health data processing system 1. Further, associated configurable reference parameter values or configurable limits can be adjusted or preconfigured in a configuration process by the health system administrator of the health data processing system 1. Accordingly, depending on the use case, from a wider group of available health related parameters a smaller group of specific vital health related parameters $v_p$ being critical for the investigated health condition of the patient P can be preselected in a configuration process by a health system administrator of the health data processing system 1. The selected subgroup of specific vital health related parameters $v_p$ can be disease-specific. For instance, only those vital health related parameters $v_p$ are preselected from all available health related parameters which have an impact on a heart disease.

The calculation unit of the user-client device 5 of the health investigating user is used to determine in step S2 an extent of deviations of parameter values of the predefined selected group of vital health related parameters $v_p$ within each loaded patient data record PDREC of a patient P from reference parameter values. These reference parameter values can comprise configurable set parameter values or configurable limits of parameter value ranges of the vital health related parameters $v_p$. The set parameter values of the vital health related parameters $v_p$ can for instance comprise adjustable threshold values for parameter value range limits derived from default settings of medical devices 3 used to collect patient data of health related parameters p stored in patient data records PDRECs of the patients P.

In a possible embodiment, for performing a medical investigation, the user-client device 5 of an authenticated health investigating user is logged-in for getting access to the parameter values of vital health related parameters $v_p$ included in the patient data records PDRECs of patients P forming part of the selected group of patients P assigned to the client device 5 of the authenticated health investigating user U and can be stored in the transitionary database 2 of the health data processing system 1. For instance, a user U1 being a cardiologist can log-in to get access to the parameter values of vital health related parameters $v_p$ included in the patient data records PDRECs of patients P assigned to him wherein the patient data records PDRECs can be downloaded from the transitionary database 2 of the health data processing system 1 by the client device 5-1 of the authenticated cardiologist U1. The patient data records PDRECs may include patient data comprising measured parameter values of a variety of different vital and/or non-vital health related parameters but also metadata of medical devices 3 used for the measurements of the health related parameters during investigations. Moreover, the patient data records PDRECs can also comprise personal data of the respective patients P, in particular a name or a reference number of the patients P.

The sorted patient record list SPRL is displayed on the graphical user interface GUI of the client device 5 of the health investigating user such as the cardiologist U1 shown in Fig. 1. The measured parameter values of the health related parameters can comprise for instance image data and/or audio data captured by sensors of medical devices 3-i. The measured parameter values of vital health related parameters $v_p$ of a patient data record PDREC displayed on the graphical user interface GUI of the client device 5-i of the health investigating user U can be displayed to the health investigating user U in a possible implementation in a pop-up window if the respective patient data record PDREC listed in the sorted patient record list SPRL is selected by the health investigating user or cardiologist U1. For instance, if the cardiologist U1 clicks by means of a user mouse on a patient P listed in the sorted patient record list SPRL, the measured parameter values of the vital health related parameters $v_p$ of the respective patient data record PDREC of this patient P are automatically displayed on the graphical user interface GUI of the client device 5 of the cardiologist U1 in a pop-up window.

Furthermore, if the total criticality metric P-CM of a patient P calculated by the processor of the client device 5-i of the health investigating user U does indicate an acute critical health condition of the respective patient P, the generation of a corresponding warning message can be automatically triggered and displayed on the graphical user interface GUI of the local client device 5 of the health investigating user U or can be transmitted in a messaging subsystem of the health data processing system 1 to one or more remote client devices 5-i of other users, in particular to other health investigating users such as physicians, cardiologists or radiologists. For instance, if the calculated total criticality metric P-CM of a patient P indicates an acute critical health condition, a warning message is not only displayed on the display unit of the Graphical user interface GUI of the client device 5-i of the investigating cardiologist but also to other users or persons using the health data processing system 1 according to the present invention. For instance, another user performing a necessary treatment of the diagnosed critical health condition can start immediately preparations to attend the affected patient P after the client device 5-j of the other user receives through the messaging subsystem of the health data processing system 1 the warning message from the client device 5-i of the health investigating user, e. g. the cardiologist U1 shown in Fig. 1. This may for instance include the preparation of a necessary surgery of the affected patient P. The generated warning message may trigger further processes within an organization such as assigning a bed to the affected patient P within a hospital. The warning message may also trigger a transport of the affected patient P from his current location to a destination such as a hospital. Moreover, if the total criticality metric P-CM of a patient P calculated by the processor of the client device 5 exceeds a configurable threshold, the patient data records PDRECs of the investigated patient P may be automatically supplied to another health investigating user such as a second cardiologist U2 for additional data analysis of the patient data included in the patient data records PDRECs of the patient P having a critical health condition.

In a possible embodiment, the parameter values of the health related parameters can also be measured by portable or by not portable medical devices 3-i of the system 1. The parameter values of the health related parameters can also be measured by sensors of wearable medical devices 3-i attached to the patients P or carried by the patients P. This allows for a remote control of a plurality of patients P participating in the health data processing system 1 according to the present invention. A plurality of patient data records PDRECs of a plurality of patients P including measured parameter values of health related parameters p can be collected in this way by a plurality of distributed medical devices 3 during investigations of the patients P over time. In a possible embodiment, a scheduler can be adapted to process a plurality of patient data records PDRECs to extract parameter values of vital health related parameters $v_p$ pre-selected from available health related parameters by a health system administrator of the health data processing system 1 and to store the extracted parameter values of the vital health related parameters $v_p$ in the transitionary database 2 of the health data processing system 1 as illustrated schematically in Fig. 1.

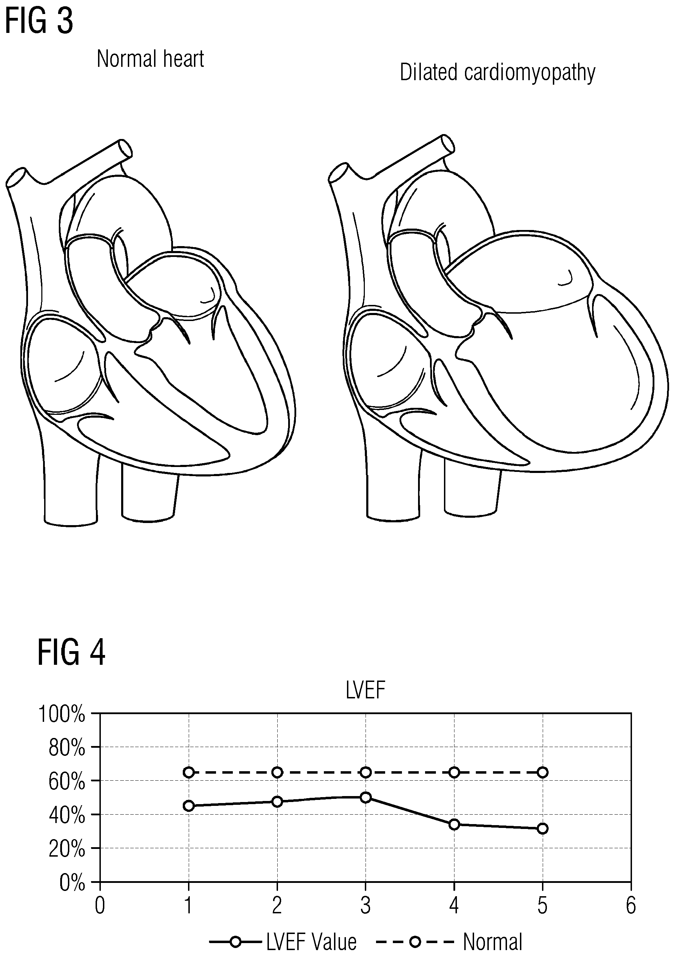

Fig. 3 illustrates a condition of a normal heart of a patient P in contrast to a heart problem classified as dilated cardiomyopathy. For example, the LVEF (ejection fraction of left ventricular) value is one of critical attributes in the commonly known heart problem classified as dilated cardiomyopathy where the left ventricular chamber size of the heart is bigger than a normal size. As a consequence, the pumping capability of the blood to the body parts of the body of the patient P is reduced and the blood oxygen concentration is low. During the course of treatment periods of the patient P, this particular health related parameter can be assessed during each investigation. Further, prior investigation values of the health related parameter can be compared by the health investigating user such as a cardiologist U to conclude whether the heart condition of the investigated patient P has improved or not. Like the LVEF health related parameter, there can be a lot of health related parameters used to assess the heart condition of the patient P during an investigation. A normal left ventricular ejection fraction (LVEF) ranges for instance between 55% to 70%. An LVEF value of 65% means for example that 65% of the total amount of blood in the left ventricle is pumped out with each heartbeat of the patient's heart.

Fig. 4 shows a diagram for illustrating the range of the LVEF health related parameter over several investigations or visits.

Investigation 1:  LVEF value = 45%
Investigation 2:  LVEF value = 48%
Investigation 3:  LVEF value = 41%
Investigation 4:  LVEF value = 35% and
Investigation 5:  LVEF value = 32%

Fig. 4 illustrates the deviation of the LVEF values from a normal value during the different Investigations I1 to I5. As can be seen in the simple example of Fig. 4, at Investigation I3, the patient P comprises an LVEF value closest to the normal healthy value. The LVEF normal value of e.g. around 65% can comprise a configurable reference parameter value used to determine deviations in step S2 of the computer-implemented method according to the present invention. Fig. 4 shows a diagram for a single vital health related parameter $v_p$. For each vital health related parameter $v_p$ which may be preconfigured as being relevant for a specific kind of investigation, a corresponding vital parameter criticality metric vp-cm can be calculated. For instance, a disease-specific group of vital health related parameters $v_p$ may comprise a first vital health related parameter $v_{p1}$ such as the LVEF parameter illustrated in Fig. 4 and another second health

related parameter $v_{p2}$ being also relevant for the investigated disease.

For each of the two health related parameters, an associated criticality metric vp-cm can be calculated automatically depending on the determined extent of deviations of the measured parameter values of the respective vital health related parameter $v_p$, from an associated reference parameter value such as the set value of 65% illustrated in Fig. 4. After having calculated the different vital parameter criticality metrics vp-cm for all vital health related parameters $v_p$ relevant for the investigated specific disease, these vital parameter criticality metrics vp-cm can be processed and aggregated to calculate a total patient criticality metric P-CM indicating a total criticality of a health condition of an investigated patient P.

$$P\text{-}CM = F\ (vp\text{-}cm1,\ vp\text{-}cm2,\ ....vp\text{-}cmN\ )$$

For instance, if only one vital health related parameter $v_p$ shows relevant deviations but all other vital health related parameters $v_p$ are within a normal range, the total criticality P-CM of the health condition of the investigated patient P can still be low. In contrast, if all vital parameter criticality metrics vp-cm of all relevant vital health related parameters $v_p$ are high indicating a high extent of deviations from a normal reference parameter value, the total patient criticality metric P-CM of the investigated patient P is high and indicates a problematic health condition of the investigated patient P. Different kinds of linear or non-linear functions F can be used to aggregate the vital parameter criticality metrics vp-cm of the different vital health related parameters $v_p$ into a single total patient criticality metric P-CM. The used aggregation function F can be preconfigured in a possible embodiment by an authenticated health system administrator of the health data processing system 1 using a configuration terminal of the health data processing system 1 depending on a type of the investigated health condition or disease and/or depending on type of a medical study.

The computer-implemented method and system 1 according to the present invention enables a health investigating user such as a cardiologist to evaluate patient data sorted in a patient record list SPRL so that he can immediately focus on those patients P having a critical health condition, in particular a critical heart condition. A sorted list of critical patients P can be automatically prepared by the computer-implemented method based on the predefined vital health related parameters $v_p$. These elected vital health related parameters $v_p$ can be preconfigured by a health system administrator or expert as contributing to the criticality of the investigated health condition of the patient P.

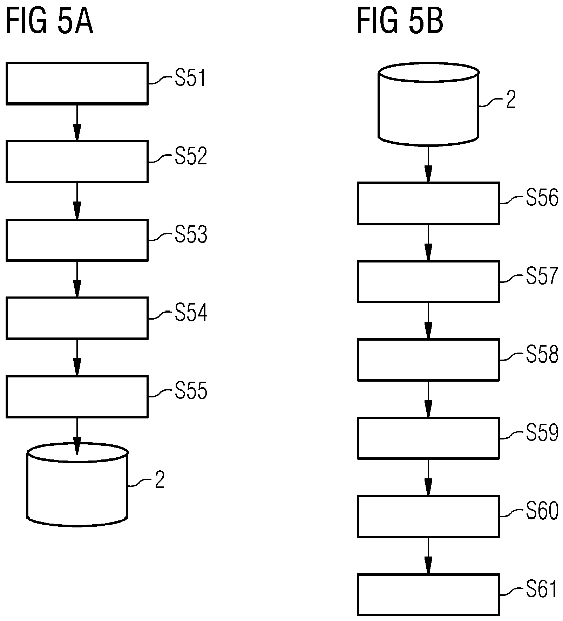

The computer-implemented method can be implemented as illustrated schematically in Figs. 5A, 5B. In a first process, the configuration of the parameters is read and the process crawls through the plurality of patient data records PDRECs stored in the transitionary database 2 of the health data processing system 1. The process gathers the parameters of the critical or vital health related parameters $v_p$ which may be predefined by a prior configuration process by a health system administrator. The parameter values of the vital health related parameters $v_p$ gathered in this way are then stored into the transitionary database 2 for later processing. This first process illustrated in Fig. 5A can be scheduled to run at low load periods, for instance every night since the process of Fig. 5A is a resource-heavy process, i.e. a process requiring extensive computing resources of the health data processing system 1.

The second process illustrated in Fig. 5B does read the patient data from the transitionary database 2 of the health data processing system 1 and may group the data logically per patient P for a further analysis. Depending on the involved health investigating user such as the cardiologist who has logged through his client device 5 into the health data processing system 1, the process illustrated in Fig. 5B can load patient data records PDRECs of each patient P forming part of a selected group of patients having been assigned to the respective health investigating user such as a cardiologist into a transitionary data structure. For this, the process can compare vital health related parameters $v_p$ and their associated preconfigured thresholds from factory definitions of the medical devices 3-i. Based on the determined variations of the values of the vital health related parameters $v_p$ from reference parameter values, an order of the patient data records PDRECs of the patients P can be changed, i.e. patients P where the vital health related parameters $v_p$ have varied the most from the reference parameter values can be automatically listed on the top of a sorted patient record list SPRL whereas the patients P having the lowest variations of the vital health related parameters $v_p$ from the reference parameter values are automatically placed at the end of the sorted patient record list SPRL. The second process illustrated in Fig. 5B can also be used to populate a secondary data structure with the parameter values. The secondary data structure can then be used to show a pop-up window W during data analysis of the patient data record PDREC within the overall sorted patient study list displayed on the graphical user interface GUI of the client device 5 of the health investigating user U. The process offers the benefit to the health investigating user U or cardiologist that he gets an immediate insight to patient data without going through the entire details of the patient data records PDRECs. In this way, the patient data of a plurality of patients P is analysed much more efficiently by the health investigating user on the basis of the generated sorted patient record list SPRL.

As illustrated in Fig. 5A, in a first step S51, a software tool such as a study list optimizer can be installed by a health system administrator of the health data processing system 1 on a client device 5 of a health investigating user U working for instance in an organization such as a hospital.

In a further step S52, the vital health related parameters $v_p$ can be preconfigured or selected by the health system administrator depending on the use case. For instance, a group of disease-specific and/or region-specific or investigation-specific vital health related parameters $v_p$ being critical for a health condition of a patient P can be selected from all available health related parameters in a configuration process by the health system administrator of the health data processing system 1 in step S52. Further, in step S53, associated configurable reference parameter values for configurable limits can be adjusted or reconfigured by the health system administrator of the health data processing system 1.

Further, scheduled downtimes or low load processing times can be set where the pre-processing of the patient data records of a plurality of patients P can be performed. For instance, a pre-processing of the plurality of raw patient data can be performed in step S54 every night. The scheduler can evaluate or process all available patient data records PDRECs generated by a plurality of heterogeneous data sources to extract in step S55 the parameter values of the vital health related parameters $v_p$ and store them in the transitionary database 2 of the health data processing system 1 as illustrated schematically in Fig. 5A.

As illustrated in the workflow of Fig. 5B, a health investigating user U such as a cardiologist can in a separated decoupled process log-in in step S56 into the health data processing system 1 to get access to the patient data stored in the transient database 2 for investigating the health condition of a group of patients P assigned to the respective health investigating user U. Execution of a second scheduler can be triggered in step S57. The second scheduler can read from the transient database 2 all the patient data records PDRECs associated with the patients P assigned to the respective health investigating user U or cardiologist in step S58. The second scheduler can further group the patient data records PDRECs logically in step S59 and can populate the corresponding data structure. In a further step S60, a scheduler may determine an extent of deviations of parameter values of all predefined vital health related parameters $v_p$ within each loaded patient data record PDREC of a patient from reference parameter values. Also weights based on the extent of deviation for each vital parameter can be calculated to arrive at the final criticality metric, thus the ordering index value. For each vital health related parameter $v_p$, an associated vital parameter criticality metric vp-cm can be calculated by a processor of the client device 5 of the health investigating user U depending on the determined extent of deviations of the measured parameter values of the respective vital health related parameter $v_p$ from the associated reference parameter values in step S60. Moreover, the determined vital parameter criticality metrics vp-cm of all vital health related parameters $v_p$ can be processed by the processor of the client device 5 of the health investigating user U to calculate a total patient criticality metric P-CM indicating a total criticality of the health condition of the investigated patient P. The patient list indices used for sorting the loaded patient data records of the selected group of patients P assigned to the health investigating user U can be automatically derived from the calculated total patient criticality metrics P-CM of the respective group of patients in step S60. These patient list indices can be used for sorting the loaded patient data records of the group of patients P assigned to the health investigating user U to generate automatically a sorted patient record list SPRL displayed on a graphical user interface GUI of the client device 5 of the health investigating user to assist in performing a data analysis of the patient data more efficiently and more focused on critical patients P.

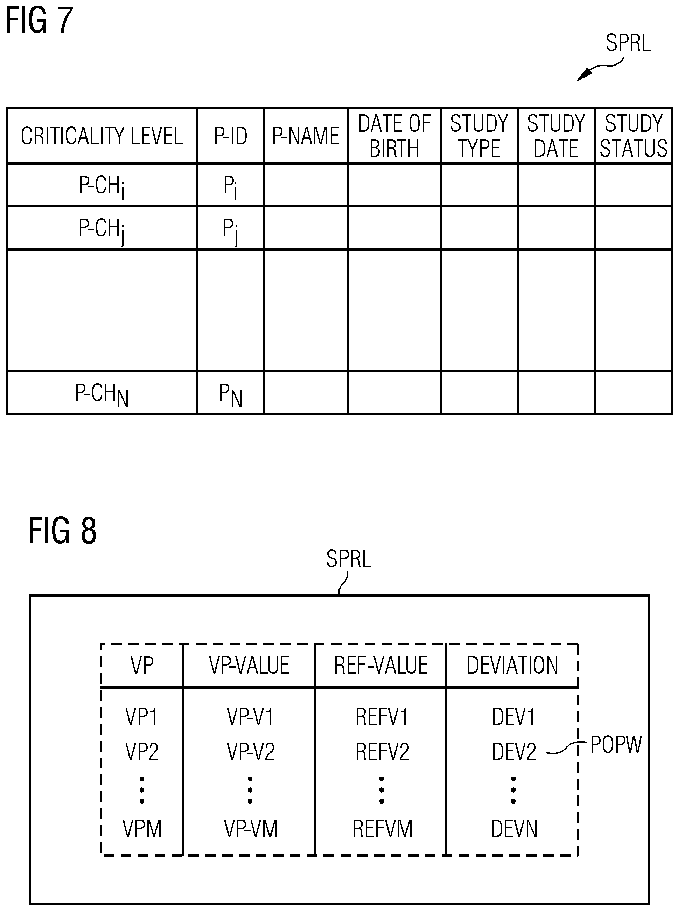

In a further step S61, the sorted list of loaded patient data records of a predefined group of patients P assigned to the health investigating user U is displayed and additional processes can be triggered depending on the parameter values of the vital health related parameters $v_p$. For instance, a corresponding warning message can be automatically triggered and displayed on the graphical user interface GUI of the client device 5 of the health investigating user if the calculated total criticality metric P-CM of a patient P calculated by the processor of the client device 5 of the health investigating user U does indicate an acute critical health condition of the respective patient P. Moreover, a pop-up window can be displayed showing parameter values of critical health related vital parameters $v_p$ as also illustrated in Fig. 8.

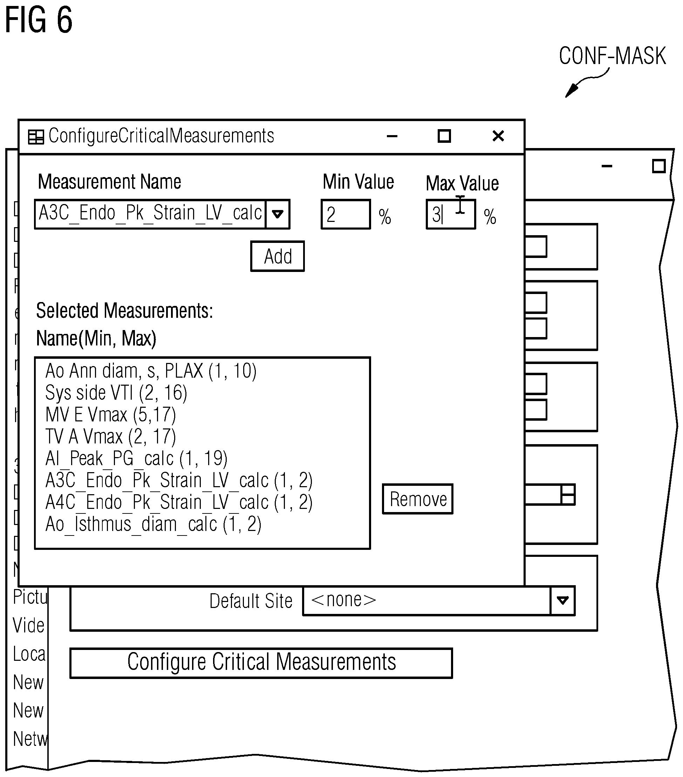

Fig. 6 shows a configuration mask which can be used in an exemplary implementation of the health data processing system 1 by a health system administrator to preconfigure vital health related parameters $v_p$ and/or to preconfigure reference parameter values including configurable limits of parameter value ranges or vital health related parameters $v_p$. The configuration process performed by the health system administrator using the configuration mask CONF-MASK displayed on a display unit of a configuration terminal of the health system administrator as illustrated in Fig. 6 includes the selection of vital health related parameters $v_p$ from all available health related parameters p and moreover a definition of configurable limits or admissible ranges for the respective selected vital health related parameters $v_p$ as illustrated in Fig. 6. For instance, in the input configuration mask CONF-MASK of Fig. 6, a health system administrator can define an admissible parameter value range between a minimum value and a maximum value. For instance, the health system administrator may predefine for a specific medical study or for a specific health condition of interest a set of vital health related parameters $v_p$ and can further define the associated admissible parameter value ranges for each parameter of the defined set of vital health related parameters $v_p$. A preselected set of vital health parameters can also be downloaded from a repository storing a set of vital health related parameters for different health conditions or study topics.

Fig. 7 shows an exemplary sorted patient record list SPRL which can be displayed on a graphical user interface GUI of a client device 5 to a health investigating user U such as a cardiologist.

At the top of the sorted patient record list SPRL are those patients P having a critical health condition, i.e. those patients P comprising a high calculated total patient criticality metric P-CM. At the bottom of the sorted patient record list SPRL are those patients P comprising a low calculated total patient criticality metric P-CM. Accordingly, the health investigating user U such as a cardiologist can immediately concentrate or focus on those patients P whose data are displayed on the top of the sorted patient record list SPRL. For example, when the health investigating user U such as a cardiologist does hover a mouse of a selection unit over the displayed patient record a pop-up window PWOW can appear as illustrated in Fig. 8 showing the parameter values of all vital health related parameters $v_p$ of the respective patient P under investigation. This reduces additional clicks and eases the scrolling across critical patients P without having to click on each displayed patient data record. The pop-up window POPW can also be color-coded to encode any critical deviations from reference values.

Further the sorted patient data records on top of the sorted patient record list SPRL can be automatically prioritized for further data processing by medical software tools adapted to perform specific medical analysing algorithms on basis of the patient data records. The sorted patient data records on top of the sorted patient record list SPRL are transmitted first by the client device 5-i of the investigating user U to other computing resources of the health data processing system 1 running specific medical software tools for further processing of the received patient data records. These medical software tools can be executed on the client device 5-i of the investigating user U and/or on client devices 5-j of other investigating user U and/or on a server of the health data processing system 1.

The selection of a matching suitable medical software tool can be performed in a possible embodiment automatically depending on the type of the investigated specific health condition and the associated configured set of vital health related parameters and corresponding data formats and/or depending on the calculated patient criticality metrics P-CM of the investigated patients P.

The data analysis can in possible embodiment be performed or supported by a diagnostic medical software tool used to perform data analysis of the patient data by executing a programmed data analysing algorithm. The medical software tool can be executed on the client device 5 of the health investigating user U or on server connected to the client device 5 of the health investigating user U though a data network or cloud 4.

**Claims**

1.    A computer-implemented method for analysing efficiently patient data of patients comprising the steps of:

loading (S1) by a user-client device (5) of a health investigating user (U) at least one patient data record, PDREC, of each patient (P) forming part of a selected group of patients (P) assigned to the client device (5) of the respective health investigating user (U) for medical investigation of the assigned group of patients (P) from a transitionary database (2) of a health data processing system (1), wherein each loaded patient data record, PDREC, of a patient (P) of said assigned group of patients comprises measured parameter values of a selected group of predefined vital health related parameters, vp, measured for the respective patient (P) by means of medical devices (3) during previously performed medical investigations of the respective patient (P);

determining (S2) by a processor of the user-client device (5) of the health investigating user (U) an extent of deviations of parameter values of the predefined vital health related parameters, vp, within each loaded patient data record, PDREC, of a patient from reference parameter values; and

sorting (S3) by the processor of the user-client device (5) of the health investigating user (U) the loaded patient data records, PDRECs, of the group of patients (P) assigned to the health investigating user (U) according to patient list indices to generate automatically a sorted patient record list, SPRL, of the assigned patients (P) depending on the determined extent of deviations of the predefined vital health related parameters, vp, and/or depending on a determined number of vital health related parameters falling out of range for performing a data analysis of patient data included in the patient data records, PDRECs, of the generated sorted patient record list, SPRL.

2.    The computer-implemented method according to claim 1 wherein the reference parameter values comprise configurable set parameter values or configurable limits of parameter value ranges of the vital health related parameters, vp, and/or comprise benchmark parameter values of the vital health related parameters, vp, derived from parameter values of vital health related parameters, vp, read from other loaded patient data records, PDRECs, of the same patient.

3.    The computer-implemented method according to claim 1 or 2 wherein a group of disease specific and/or region specific and/or investigation specific vital health related parameters, vp, being critical for a health condition of a patient (P) is selected from available health related parameters, p, and associated configurable reference parameter values or configurable limits are adjusted or reconfigured in a configuration process by a configuration terminal of a health system administrator of the health data processing system (1).

4. The computer-implemented method according to any of the preceding claims 1 to 3, wherein for each vital health related parameter, vp, a vital parameter criticality metric, vp-cm, is automatically determined by a processor of the client device (5) of the health investigating user (U) depending on the determined extent of calculated deviations of the measured parameter values of the respective vital health related parameter, vp, from the associated reference parameter values.

5. The computer-implemented method according to claim 3 or 4, wherein the determined vital parameter criticality metrics, vp-cm, of all vital health related parameters, vp , are processed by the processor of the client device (5) of the health investigating user (U) to calculate a total patient criticality metric, P-CM, indicating a total criticality of a health condition of an investigated patient (P).

6. The computer-implemented method according to claim 5, wherein the patient list indices used for sorting the loaded patient data records, PDRECs, of the selected group of patients (P) assigned to the client device (5) of the health investigating user (U) are derived from the calculated total patient criticality metrics, P-CM, of the respective group of patients.

7. The computer-implemented method according to any of the preceding claims 1 to 6,
   wherein the set parameter values of the vital health related parameters, vp, comprise adjustable threshold values for parameter value range limits derived from default settings of medical devices used to collect patient data of health related parameters, p, stored in patient data records, PDRECs, of patients.

8. The computer-implemented method according to any of the preceding claims 1 to 7,
   wherein for performing a medical investigation the user-client device (5)of an authenticated health investigating user is logged-in for getting access to the parameter values of vital health related parameters, vp, included in the patient data records, PDRECs, of patients (P) forming part of a selected group of patients assigned to the client device (5) of the authenticated health investigating user (U) and being stored in the transitionary database (2) of the health data processing system (1).

9. The computer-implemented method according to any of the preceding claims 1 to 8, wherein the patient data records, PDRECs, include patient data comprising measured parameter values of a variety of different vital and non-vital health related parameters, metadata of medical devices (3) used for the measurements of the health related parameters during investigations and personal data of the plurality of patients.

10. The computer-implemented method according to any of the preceding claims 1 to 9,
    wherein the generated sorted patient record list, SPRL, is displayed on a graphical user interface, GUI, of the client device (5) to the health investigating user (U).

11. The computer-implemented method according to any of the preceding claims 1 to 10 wherein measured parameter values of vital health related parameters, vp, of a patient data record, PDREC, displayed on the graphical user interface, GUI, of the client device (5) of the health investigating user (U) are displayed to the health investigating user (U)in a pop-up window (POPW)if the respective patient data record, PDREC, listed in the sorted patient record list, SPRL, is selected by a selection command input unit connected to the client device (5) of the health investigating user (U).

12. The computer-implemented method according to any of the preceding claims 1 to 11 wherein the data analysis of patient data included in selected patient data records, PDECs, of the generated sorted patient record list, SPRL, is performed by means of at least one matching medical software tool executed on a client device (5)of a health investigating user (U) and/or by a server of the health data processing system (1)

13. A client device (5) of a health investigating user (U) comprising:

    a data interface used to load (S1) at least one patient data record, PDREC, of each patient (P) forming part of a selected group of patients (P) assigned to a client device (5)of the respective health investigating user (U)for medical investigation of the assigned group of patients (P) from a transitionary database (2)of a health data processing system (1),

wherein each loaded patient data record, PDREC, of a patient (P) of said assigned group of patients comprises measured parameter values of vital health related parameters, vp, measured for the respective patient (P) by means of medical devices (3) during previously performed medical investigations of the respective patient (P); and

a data processing unit configured to determine (S2) an extent of deviations of parameter values of a selected group of predefined vital health related parameters, vp, within each loaded patient data record, PDREC, of a patient (P) from reference parameter values, and configured to sort (S3) the loaded patient data records, PDRECs, of the group of patients assigned to the client device (5) of the health investigating user (U) according to patient list indices to generate automatically a sorted patient record list, SPRL, of the assigned patients depending on the determined extent of deviations of the predefined vital health related parameters, vp, said sorted patient record list, SPRL being used for performing a prioritized data analysis of patient data included in the patient data records, PDRECs, of the generated sorted patient record list, SPRL, displayed on a graphical user interface, GUI, of the client device (5) to the health investigating user (U).

14. A health data processing system (1) comprising client devices (5) of health investigating users (U), in particular physicians, having access to a transitionary database (2) of the health data processing system (1), wherein the client devices (5) are adapted to perform the computer-implemented method according to any of the preceding claims 1 to 12.

15. The health data processing system according to claim 14, wherein a scheduler is adapted to process the plurality of patient data records, PDRECs, to extract automatically parameter values of vital health related parameters, vp, selected from available health related parameters, p, through a configuration mask displayed to a terminal of a health system administrator of the health data processing system (1) and adapted to store the extracted parameter values of the vital health related parameters, vp, in the transitory database (2) of the health data processing system (1).

# FIG 1

# FIG 2

# FIG 3

Normal heart

Dilated cardiomyopathy

# FIG 4

LVEF

LVEF Value    Normal

# FIG 5A

# FIG 5B

# FIG 6

CONF-MASK

⊞ ConfigureCriticalMeasurements  — ☐ ✕

Measurement Name        Min Value    Max Value

A3C_Endo_Pk_Strain_LV_calc ▾    2  %    3  %

Add

Selected Measurements:
Name(Min, Max)

Ao Ann diam, s, PLAX (1, 10)
Sys side VTI (2, 16)
MV E Vmax (5,17)
TV A Vmax (2, 17)
AI_Peak_PG_calc (1, 19)
A3C_Endo_Pk_Strain_LV_calc (1, 2)
A4C_Endo_Pk_Strain_LV_calc (1, 2)
Ao_Isthmus_diam_calc (1, 2)

Remove

Pictu
Vide
Loca
New
New
Netw

Default Site  <none>  ▾

Configure Critical Measurements

# FIG 7

SPRL

| CRITICALITY LEVEL | P-ID | P-NAME | DATE OF BIRTH | STUDY TYPE | STUDY DATE | STUDY STATUS |
|---|---|---|---|---|---|---|
| P-CH$_i$ | P$_i$ | | | | | |
| P-CH$_j$ | P$_j$ | | | | | |
| | | | | | | |
| P-CH$_N$ | P$_N$ | | | | | |

# FIG 8

SPRL

| VP | VP-VALUE | REF-VALUE | DEVIATION |
|---|---|---|---|
| VP1 | VP-V1 | REFV1 | DEV1 |
| VP2 | VP-V2 | REFV2 | DEV2 |
| ⋮ | ⋮ | ⋮ | ⋮ |
| VPM | VP-VM | REFVM | DEVN |

POPW

Europäisches
Patentamt

European
Patent Office

Office européen
des brevets

# EUROPEAN SEARCH REPORT

Application Number

EP 22 17 2780

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/216556 A1 (MARTIN NEIL [US] ET AL) 27 August 2009 (2009-08-27) * paragraphs [0013] - [0016], [0044] - [0046], [0050], [0051], [0057], [0058], [0107], [0115], [0116], [0123], [0129], [0132]; figures 1-6, 9b, 17, 19, 22 * | 1-15 | INV. G16H10/60 G16H50/30 G16H40/20 |
| X | US 2014/222446 A1 (ASH MICHAEL ALAN [US] ET AL) 7 August 2014 (2014-08-07) * paragraphs [0004], [0005], [0017], [0034] - [0036], [0038], [0057], [0059], [0062], [0063], [0065], [0068], [0077] - [0079]; figures 3,4,7 * | 1-15 | |
| X | US 2020/160998 A1 (WARD KEVIN R [US] ET AL) 21 May 2020 (2020-05-21) * paragraphs [0005], [0006], [0031], [0046], [0047], [0072] - [0074]; figures 2A, 5A, 5B, 6 * | 1-15 | |

TECHNICAL FIELDS SEARCHED (IPC)

G16H

The present search report has been drawn up for all claims

| Place of search | Date of completion of the search | Examiner |
|---|---|---|
| Munich | 18 October 2022 | Hendrikse, Natalie |

EPO FORM 1503 03.82 (P04C01)

## ANNEX TO THE EUROPEAN SEARCH REPORT
## ON EUROPEAN PATENT APPLICATION NO.

EP 22 17 2780

This annex lists the patent family members relating to the patent documents cited in the above-mentioned European search report.
The members are as contained in the European Patent Office EDP file on
The European Patent Office is in no way liable for these particulars which are merely given for the purpose of information.

18-10-2022

| Patent document cited in search report | | Publication date | Patent family member(s) | | Publication date |
|---|---|---|---|---|---|
| US 2009216556 | A1 | 27-08-2009 | CA | 2654679 A1 | 24-08-2009 |
| | | | EP | 2093683 A2 | 26-08-2009 |
| | | | JP | 2009219867 A | 01-10-2009 |
| | | | US | 2009216556 A1 | 27-08-2009 |
| US 2014222446 | A1 | 07-08-2014 | NONE | | |
| US 2020160998 | A1 | 21-05-2020 | NONE | | |

EPO FORM P0459

For more details about this annex : see Official Journal of the European Patent Office, No. 12/82